# EUROPEAN PATENT APPLICATION

(11) **EP 2 534 959 A1**
(43) Date of publication of application: **19.12.2012**
(21) Application number: 11169834.6
(22) Date of filing: 14.06.2011
(51) Int. Cl.: A23L 1/10, A23L 1/164, A23L 1/168, A23L 1/30, A61K 36/899, A61K 36/8998

(54) **Wholegrain cereal and liver disorders**

(71) Applicant: Nestec S.A., 1800 Vevey (CH)
(72) Inventor: Ross, Alastair, 1800 Vevey (CH); Godin, Jean-Philippe, 1024 Ecublens (CH); Bruce, Steve, 1066 Epalinges (CH); Rezzi, Serge, 1623 Semsales (CH); Guy, Phlippe, 1000 Lausanne 25 (CH)
(74) Representative: Marquardt, Ulf

(57) **Abstract**

The present invention relates generally to the field of compositions containing wholegrain cereals. In particular, the present invention relates to health benefits of wholegrain cereal. One embodiment of the present invention concerns a composition comprising wholegrain cereal for use in the treatment or prevention of liver disorders.

## Description

The present invention relates generally to the field of compositions containing wholegrain cereals. In particular, the present invention relates to health benefits of wholegrain cereals. One embodiment of the present invention concerns a composition comprising wholegrain cereals for use in the treatment or prevention of liver disorders.

Liver disorders occur widely in the population and are a risk factor for early mortality and more serious liver diseases.

For example, fatty liver disease (FLD) exists in approximately 10-24 % of the population, depending on the country, and may have a prevalence of about 75 % among obese people. One German study estimated that having fatty liver disease increased a patients health costs for the Government by 26 % over a 5 year period (Baumeister SE, et al. (2008) Gastroenterology 134: 85-94).

Fatty liver disease (FLD) is an inflammation of the liver, caused by the build up of fat in the liver cells which can over time lead to decreased liver function and if untreated, cirrhosis (where the liver ceases to function properly). When seen under a microscope slide, fatty liver cells have large apparently empty spaces, which are fat deposits. This fat accumulation ultimately pushes the cell nucleus to the edge of the cell, and the cell ceases to function normally. The reasons for the increase in fat accumulation are widely debated, but are suggested to involve a decrease in function of some cell signalling pathways, general insulin resistance, and/or a lack of the methyl-donor betaine. One of the possible causes of FLD is a metabolic disequilibrium around the one-carbon metabolic cycle. In addition, other metabolic factors may play a roll in the accumulation of lipids (e.g. loss of insulin sensitivity). While a better diet and exercise are the main recommended treatments, there are no specific food recommendations.

There are two main types of FLD-alcoholic FLD, where subjects regularly consume >20 g/d alcohol, and alcohol is considered to be the main cause of the fat build up; and non-alcoholic FLD (NAFLD), which is not caused by alcohol, and often related to insulin resistance and metabolic syndrome. The extreme form of NAFLD is known as non-alcoholic steatohepatisis (NASH). NASH is considered to be one of the main causes of otherwise undefined cirrhosis of the liver (over time, around 20 % of people with NASH will go on to develop cirrhosis of the liver). If treated early enough, the condition is reversible.

NAFLD is often associated with impaired insulin metabolism, obesity and metabolic syndrome. It is also caused by protein malnutrition (rapid weight loss may exacerbate the condition in obese people), and treatment with corticosteroids (e.g. cortisone and prednisone). Alcoholic-FLD is generally associated with chronic alcohol abuse, and in this case the metabolism of alcohol by the enzyme alcohol dehydrogenase also sends a signal for an increase in fatty acid synthesis and a decrease in fatty acid breakdown.

FLD is usually diagnosed using liver function tests. Alcohol consumption over 20 g/d is considered to be the threshold for considering alcohol abuse to be the cause of the FLD condition.Abnormal liver enzyme values (e.g. alanine transaminase; aspartate transaminase, bilirubin and γ-glutamyl transpeptidase), and ultrasound observation of steatosis (abnormal accumulation of fat within cells) are minimally invasive markers of FLD, but definitive diagnosis can only be made with a liver biopsy and histological examination. Current treatments of FLD usually involve weight loss, and may include the administration of metformin and thiazolidinediones. Improvements of diet and exercise habits appear to be the most effective long-term treatments. Some experimental evidence also suggests that supplementation with glycine betaine (betaine) can reverse the progression of both alcoholic and non-alcoholic FLD (Miglio F, et al. (2000) Arzneim. Forsch./Drug Research 50: 722-727; Samara K, et al. (2006) Digestive Diseases and Sciences 51: 1226-1229).

US2002/0119181A1 discloses nutritional compositions made from conventional foods mixed on-site in a blender for treating patients with hepatic disorders. Thereby, a nutritionally complete, calorically dense mixture comprising a range of vitamin and mineral rich foods is achieved. The purpose of the invention is to address and correct the malnutrition as common in many hepatic diseased patients. However, the document fails to specifically provide a treatment or prevention of the hepatic disorder itself.

US7,078,064B2 relates to compositions comprising antioxidants useful for reducing oxidative stress and lipid peroxidation and thereby treating a.o. chronic liver diseases. In particular, the document relates to compositions supplemented with antioxidants, including certain vitamins prepared from plant materials or produced by synthetic chemistry. The main route for this formulation is via parental administration, i.e. intra-venous supply of nutrients when the oral route is not possible. The document fails to provide a natural and simple oral food product solution for the treatment or prevention of liver disorders.

In view of the broad distribution of liver disorders in today's population and substantial healthcare costs it would be desirable to have available a natural composition that would be cheap and available to everyone and could be used to prevent and/or treat liver disorders and/or reverse existing liver damage.

The present inventors have addressed this need and have found that the subject matter of the independent claim achieves the object described above.

The thereon dependant claims further develop the idea of the present invention.

In particular, the inventors were able to provide a composition that can be used, e.g., as food product and would consequently be available to everybody and that can be used to treat or prevent liver disorders.

The inventors were surprised to see the administration of a wholegrain diet for 7 days results in a continuously increased plasma betaine concentration. It was also shown that the plasma betaine concentration acutely increases after a breakfast wholegrain cereal challenge.

Example 2 further substantiates the beneficial effect of wholegrain cereal in an animal study.

As supplementation with betaine has been linked to improvement in fatty liver, the results show that the compositions of the present invention improve liver stores of betaine, which will help prevent the development and progression of liver diseases.

Consequently, one embodiment of the present invention is a composition comprising wholegrain cereal for use in the treatment or prevention of liver disorders.

The present invention also related to the use of wholegrain cereal for the preparation of a composition to treat or prevent liver disorders.

For example, the composition comprises at least about 0.3 g wholegrain cereal/g dry weight.

Wholegrain cereal is to be understood for the purposes of the present invention as cereal grains that contain bran and germ as well as the endosperm.

Refined cereal contains essentially only the endosperm.

For the purposes of the present invention wholegrain shall be understood as proposed by the guidelines of the United States Food and Drug Administration follows:
"Cereal grains that consist of the intact, ground, cracked or flaked caryopsis, whose principal anatomical components - the starchy endosperm, germ and bran - are present in the same relative proportions as they exist in the intact caryopsis - should be considered a wholegrain...
Cereal grains may include amaranth, barley, buckwheat, bulgur, corn (including popcorn), millet, quinoa, rice, rye, oats, sorghum, teff, triticale, wheat, and wild rice (Guidelines of the United States Food and Drug Administration).

Advantageously, wholegrain cereal is an ingredient which is generally well accepted and liked by consumers. It is a natural ingredient that can be incorporated into a large variety of products, and consequently, will be available to everyone in sufficient quantities.

Moreover, the composition of the present invention does not require the addition of purified chemical compounds, which may require a careful dosing, and which may have undesirable side effects.

Advantageously, the composition of the present invention may be administered in an amount that corresponds to a consumption of at least about 30g wholegrain cereal per day. Preferably, the amount to be administered corresponds to a consumption of at least about 100g wholegrain cereal per day.

While in principle any amount of wholegrain cereal will have a positive effect on liver health following a dose response curve, good effects are seen if at least 30g wholegrain cereal are consumed per day, and even better effects are achieved by administering at least about 100g wholegrain cereal per day. The preferred embodiment is an administration of a composition that corresponds to a daily consumption of wholegrain cereal of about 160g. Such amounts of wholegrain cereal consumption can be feasibly achieved when for example a part or most of the dietary carbohydrates are based on wholegrain cereals. Excessive consumption of wholegrain cereal on the other hand is likely not to produce further increasing positive effects on liver health, but rather a saturation effect must be expected. Hence, in one embodiment the composition is to be administered in an amount corresponding to a consumption of between about 30-200g wholegrain cereal per day.

The wholegrain cereal may be formulated in any kind of composition. However, such wholegrain compositions are generally perceived as pleasant to consume if they contain fibre, carbohydrates, fat and protein in a specific ratio. For example, the composition of the present invention may contain about 3-20 weight-% fibre, about 60-85 weight-% carbohydrates, about 0.5-10 weight-% fat and about 5-15 weight-% protein.

Optionally, the composition of the present invention may also contain vitamins, minerals, trace elements and other micronutrients, e.g., in accordance with the recommendations of Government bodies such as the USRDA.

For example, the composition may contain per daily dose one or more of the following micronutrients in the ranges given: 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 µg iodine, 5 to 15 µg selenium, 1000 to 3000 µg beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 µg Vitamin B12, 100 to 800 µg folic acid, 30 to 70 µg biotin, 1 to 5 µg Vitamin D, 3 to 10 µg Vitamin E.

The composition may also contain further compounds that are known to have a beneficial effect for the liver.

For example, for the provision of an immediate effect the composition may further comprise added betaine (N,N,N-trimethylglycine). Betaine may be added as purified betaine or as a composition with a high betaine concentration. This will have the advantage that the added betaine will enforce an immediate beneficial effect for the liver while the wholegrain cereal will ensure that the betaine effect will last for prolonged time periods.

As wholegrain cereal any kind of cereal or pseudocereal may be used. For example, the cereal may be selected from the group consisting of wheat, rye, triticale, oats, barley, rice, maize, sorghum, millet, quinoa, buckwheat or combinations thereof.

The wholegrain cereals may be processed, e.g., to improve storage times, the pleasure of consumption, and/or to way wholegrain can be incorporated into a final product.

Hence, the wholegrain cereals may at least in part processed.

Typically, wholegrain cereals may be processed by a method selected from the group consisting of enzyme treatments, malting, heating, extrusion, baking, cooking or combinations thereof.

The composition of the present invention may be any kind of consumable product. For example, the composition may be selected from the group consisting of food products, an animal food products, pharmaceutical compositions, nutritional compositions, nutraceuticals, drinks, or food additives.

Pharmaceutical compositions have the advantage that they are generally administered under the supervision of medical personnel, which allows the determination of exact dosage forms for the subject to be treated.

Food products, animal food products, nutritional compositions, nutraceuticals, drinks, and food additives have the advantage that they are freely available to everyone and can consequently particularly well be used as preventive measure.

Generally, the composition of the present invention can be consumed at any time.

However, it was found that the composition is in particular pleasant to consume during or as breakfast.

Alternatively, the composition of the present invention may be consumed during or as lunch or breakfast.

Without wishing to be bound by theory the inventors believe that this preference is due to the fact that it usually takes longer to digest wholegrain products than products from refined cereals. If this digestion procedure is ongoing through the night, this might be considered problematic for people that have trouble sleeping.

The composition of the invention can also be administered at least twice during the day, whereby one administration may include for example breakfast. Preferably, the composition is administered once in the morning, e.g. during or as breakfast, and once in the after-noon, for example during or as part of the lunch meal. This dieting regimen may particularly be applicable, where consumption of wholegrain cereal of 100g or more per day is envisaged. In this way, the daily consumption can be split into individual servings during the day, which allows to spread the up-take of e.g. carbohydrates and diverse beneficial phytochemicals present in the wholegrain cereal more evenly during the entire day and not to concentrate such consumption to one single meal. Further, the up-take and bioavailability of the effective nutrients from the whole grain will be more evenly spread during the day and hence have a much better and sustainable biological effect to the body of a consumer.

The liver disorder may be selected from the group consisting of fatty liver disease, non-alcoholic steatosis hepatitis, alcoholic steatosis hepatitis, liver cirrhosis, cholestasis or combinations thereof.

The composition of the present invention may be intended for humans or animals.

For animals the disorder to be treated or prevented may be fatty liver disease, non-alcoholic steatosis and/or liver cirrhosis and the composition may be formulated as a pet food composition.

Alternatively, the composition may also be formulated as a pet food snack or as a pet food additive.

If intended for humans, the composition of the present invention is particular intended for people at risk of developing a liver disorder, people suffering from liver disorders, and/or people recovering from liver disorders.

For example, the composition of the present invention may be to be administered to overweight or obese people.

"Overweight" is defined for an adult human as having a BMI between 25 and 30.

"Obesity" is a condition in which the natural energy reserve, stored in the fatty tissue of animals, in particular humans and other mammals, is increased to a point where it is associated with certain health conditions or increased mortality. "Obese" is defined for an adult human as having a BMI greater than 30.

"Body mass index" or "BMI" means the ratio of weight in kg divided by the height in metres, squared.

Consequently, the composition of the present invention may be to be administered to people with a BMI of above at least 25.

Since FLD is often caused by increased alcohol consumption, the composition of the present invention may also be to be administered to people with an above average alcohol consumption.

For example, the composition may be to be administered to subjects which consume on average at least about 20 g alcohol per day.

Of course, the composition of the present invention may also be to be administered to people suffering from alcoholism.

Since the risk of developing liver disorders increases with age, the composition may also be to be administered to people above the age of 40.

Some exemplary embodiments of the present invention are listed below:
In one embodiment the disorder to be treated or prevented is FLD, the wholegrain cereal is wheat and the composition is intended for people above the age of 40.

In a further embodiment the disorder to be treated or prevented is FLD, the wholegrain cereal is oats and the composition is intended for people above the age of 50.

In a further embodiment the disorder to be treated or prevented is FLD, the wholegrain cereal is maize and the composition is intended for people above the age of 40.

In a further embodiment the disorder to be treated or prevented is FLD, the wholegrain cereal is rice and the composition is intended for people above the age of 40.

In a further embodiment the disorder to be treated or prevented is FLD, the wholegrain cereal is wheat and the composition is intended for people with a BMI of above at least 25.

In a further embodiment the disorder to be treated or prevented is FLD, the wholegrain cereal is oats and the composition is intended for people with a BMI of above at least 25.

In a further embodiment the disorder to be treated or prevented is FLD, the wholegrain cereal is maize and the composition is intended for people with a BMI of above at least 25.

In a further embodiment the disorder to be treated or prevented is FLD, the wholegrain cereal is rice and the composition is intended for people with a BMI of above at least 25.

It is clear to those of skill in the art that they can freely combine all features of the present invention disclosed herein without departing from the scope of the invention as disclosed.

Further advantages and features of the present invention are apparent from the following example and drawing.
Figure 1 shows that plasma betaine is increased after one week on a wholegrain cereal diet.
Figure 2 shows that plasma betaine is increased after a wholegrain breakfast cereal. All points are different from the refined grain diet, and the maximum of 120 minutes is different from all other timepoints measured.

### Example 1:

In a randomised cross-over trial, subjects had one of two different energy matched diets: one based on wholegrain cereals, containing Nestlé wholegrain products (e.g. Buitoni pasta and crackers, Lean Cuisine, DON couscous, Shreddies, Shredded Wheat, Uncle Toby's muesli, porridge, barley risotto, rye bread), and the other containing refined grain cereal products (e.g. Cornflakes, Rice Krispies, Nestlé equivalent non-wholegrain cereals). The composition of the wholegrain cereal foods falls within the following macronutrient specification: 100-400 kcal/100 g; 4-15 g protein/100 g; 0.2-30 g fat/100 g and 2-25 g fibre/100 g. The average total daily intake of wholegrains on the wholegrain diet was 150 g/d (dry weight basis). With the exception of fibre, diets were matched for total energy and macronutrient content.

Eleven female and 6 male healthy subjects aged between 20 and 50 took part in the trial. Subjects followed one of the diets for two weeks, and then followed the other after at least 8 weeks of washout (normal and non-wholegrain diet). On day 8 of each controlled diet period, the subjects undertook a post-prandial challenge, where they consumed a breakfast appropriate for the diet, and then gave blood samples over the next 4 hours.

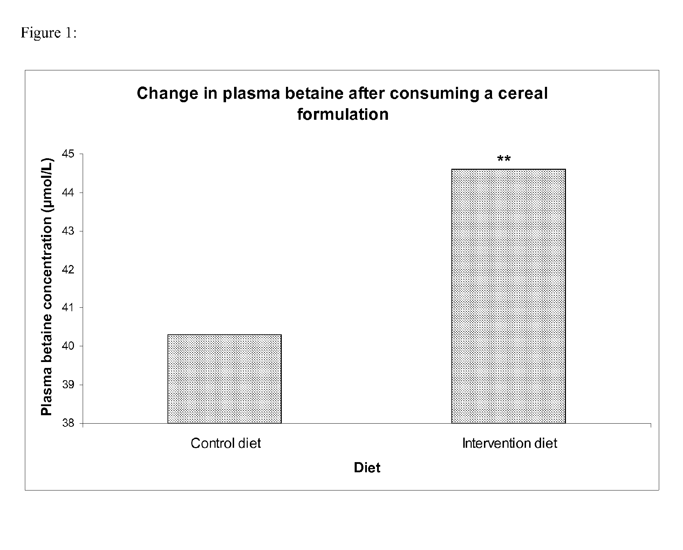

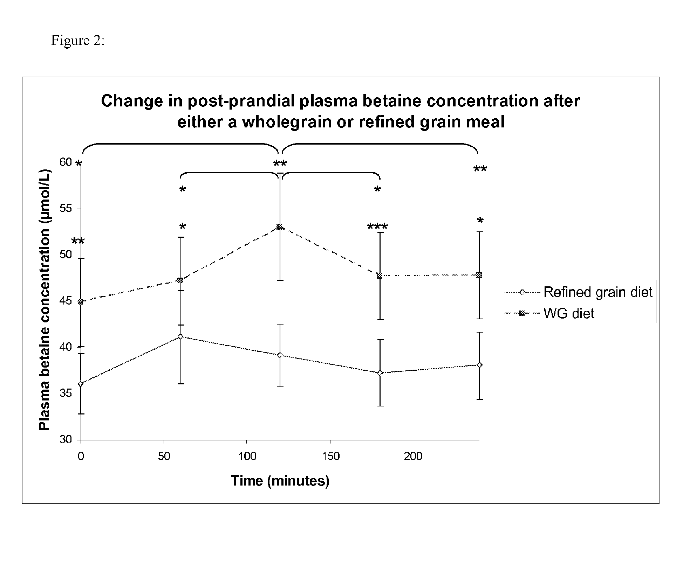

Differences between subjects were measured using a repeated measures ANOVA and a paired t-test, and were significant at the P<0.05 level. Plasma betaine concentrations increased after 7 days on the wholegrain diet (Figure 1), and increased acutely after the breakfast cereal challenge (Figure 2).

As supplementation with betaine has been linked to improvement in fatty liver, the results show that the compositions of the present invention improve liver stores of betaine, which will help prevent the development and progression of liver diseases. Other factors in wholegrain cereals may also help to improve insulin sensitivity, which could also help prevent and treat the development of, e.g., FLD.

### Example 2:

The effect of wholegrain cereal on the reduction of symptoms of fatty liver can be tested in an animal study using for example Zucker rats. Zucker (fa/fa) rats have a defect for the gene that regulates appetite, so spontaneously develop fatty liver because they overeat. One of the main risk factors for fatty liver in humans is obesity brought about by overconsumption of food. Zucker (fa/+) rats are genetically similar (littermates), but are heterozygous for this gene defect, and thus serve as normal weight controls.

The study is conducted with four groups, with each group of 10 animals aged 8 weeks at the start of the study and fed with a different diet as follows:
1) Zucker (fa/fa) rats fed a refined grain-based diet
2) Zucker (fa/fa) rats fed a wholegrain-based diet
3) Zucker (fa/fa) rats fed a refined grain-based diet, with added glycine betaine to match the diet of Group 2
4) Zucker (fa/-) rats fed a refined grain-based diet ('healthy control group')

Diets are based on the AIN-93G diet and matched for total energy, total fibre and percentage of energy coming from fat, carbohydrate and protein. 50 % of the diets by weight come from wheat flour (either refined or wholegrain). This corresponds to a total wholegrain cereal consumption of about 100g to 160g per day for an average sized adult person. All diets are supplemented with standard vitamin and mineral mixes, so that deficiencies of these should not be responsible for any differences observed.

The rats are fed ad libitum, with paired feeding after two weeks if intake between groups is different. In total rats can be fed the different diets for 8 weeks. After 8 weeks, the rats are sacrificed and liver samples taken to assess the development of fatty liver, based on liver histopathology (size of fat deposits and abundance of inflammatory cells), total liver fat content, as well as triglycerides, fatty acids and cholesterol.

## Claims

1. Composition comprising at least about 0.3 g wholegrain cereal/g dry weight for use in the treatment or prevention of liver disorders.

2. Composition in accordance with claim 1, wherein the composition is to be administered in an amount that corresponds to a consumption of at least about 30g wholegrain cereal per day.

3. Composition in accordance with one of the preceding claims, wherein the composition is to be administered in an amount that corresponds to a consumption of at least about 100g, preferably of about 160g wholegrain cereal per day.

4. Composition in accordance with one of the preceding claims, wherein the composition is to be administered in an amount corresponding to a consumption of not more than about 200g wholegrain cereal per day.

5. Composition in accordance with one of the preceding claims comprising about 3-20 weight-% fibre, about 60-85 weight-% carbohydrates, about 0.5-10 weight-% fat and about 5-15 weight-% protein.

6. Composition in accordance with one of the preceding claims, wherein the composition further comprises added betaine (N,N,N-trimethylglycine).

7. Composition in accordance with one of the preceding claims, wherein the cereal is selected from the group consisting of wheat, rye, triticale, oats, barley, rice, maize, sorghum, millet, or combinations thereof.

8. Composition in accordance with one of the preceding claims, wherein the cereals are at least in part processed.

9. Composition in accordance with claim 6, wherein the cereals are processed by a method selected from the group consisting of enzyme treatments, malting, heating, extrusion, baking, cooking or combinations thereof.

10. Composition in accordance with one of the preceding claims, wherein the liver disorder is selected from the group consisting of fatty liver disease, non-alcoholic steatosis hepatitis, alcoholic steatosis hepatitis, liver cirrhosis, cholestasis or combinations thereof.

11. Composition in accordance with one of the preceding claims, wherein the composition is selected from the group consisting of food products, an animal food products, pharmaceutical compositions, nutritional compositions, nutraceuticals, drinks, or food additives.

12. Composition in accordance with one of the preceding claims to be administered during or as breakfast.

13. Composition in accordance with one of the preceding claims to be administered at least twice a day, preferably at least once in the morning and once during lunch or in the after-noon.

14. Composition in accordance with one of the preceding claims to be administered to people with a BMI of above at least 25, to people with an above average alcohol consumption , to people suffering from alcoholism, and/or to people above the age of 40.
